# EUROPEAN PATENT APPLICATION

(11) **EP 4 108 763 A1**
(43) Date of publication of application: **28.12.2022**
(21) Application number: 21757155.3
(22) Date of filing: 16.02.2021
(51) Int. Cl.: C12N 1/20, C12P 7/52

(54) **STRAIN CAPABLE OF PRODUCING BUTYRIC ACID**

(30) Priority: 21.02.2020 JP 2020028498
(71) Applicant: Higher Mount Co., Ltd., Gifu-shi, Gifu, 500-8441 (JP)
(72) Inventor: TAKAYAMA Hirotaka, Gifu-shi, Gifu 500-8441 (JP); TAKAYAMA Emi, Gifu-shi, Gifu 500-8441 (JP)
(74) Representative: Gill Jennings & Every LLP
(86) International application number: PCT/JP2021/005685
(87) International publication number: WO 2021/166901

(57) **Abstract**

The objective of the present invention is to provide a strain that efficiently produces butyric acid and a method for producing butyric acid using the strain. The present invention provides Clostridium beijerinckii SIID27451-B11 strain (Accession No. NITE BP-02951), which produces more butyric acid and less lactic acid and acetic acid in a culture solution after anaerobically culturing the strain at 37 °C for 72 hours, compared with Clostridium beijerinckii NBRC 109359T, which is a type strain of Clostridium beijerinckii. Furthermore, the present invention provides a method for producing butyric acid using SIID27451-B11 strain

## Description

### [Technical Field]

This invention relates to a strain having a butyric acid-producing ability and a method for producing butyric acid using the strain.

### [Background Art]

In recent years, the usefulness of butyric acid has been the focus of much attention. It has become clear that butyric acid is utilized as an energy source in large intestines and may improve immune system disorders by maintaining intestinal flora in healthy state. Butyric acid bacteria (butyric acid-producing bacteria) are a general term for bacteria that produce butyric acid through metabolism. Butyric acid bacteria are a type of intestinal bacteria that produce butyric acid by decomposing dietary fiber in the intestines. Conventionally, Clostridium butyricum has been known as a representative butyric acid-producing bacterium that has been used for applications such as a medicine for intestinal regulation. However, butyric acid-producing bacteria that produce butyric acid more efficiently are needed.

Also, butyric acid is useful not only for biological applications as mentioned above, but also as a raw material for industrial applications, such as synthesis of fragrances. For such applications, butyric acid-producing bacteria that produce butyric acid more efficiently are also needed.

### [Summary of Invention]

### [Problem to be solved]

The objective of the present invention is to provide a strain that efficiently produces butyric acid and a method for producing butyric acid using the strain.

### [Solution to Problem]

In the process of producing fermented liquid containing short-chain fatty acids such as butyric acid, propionic acid, and lactic acid by fermenting various natural raw materials, such as soybeans and crude drugs, the inventors have found and isolated SIID27451-B11 strain (accession number NITE BP-02951). The SIID27451-B11 strain was identified as a strain of Clostridium beijerinckii.

Thus, the present invention provides Clostridium beijerinckii SIID27451-B11 strain (accession number NITE BP-02951).

The present invention also provides Clostridium beijerinckii SIID27451-B11 strain having genome sequences shown in sequence numbers 1 to 5.

The present invention also provides Clostridium beijerinckii SIID27451-B11 strain having a 16S rDNA sequence shown in sequence number 6.

The present invention also provides Clostridium beijerinckii SIID27451-B11 strain which produces more butyric acid and less lactic acid and acetic acid in a culture solution after anaerobically culturing the strain at 37 °C for 72 hours, compared with Clostridium beijerinckii NBRC 109359T, which is a type strain of Clostridium beijerinckii.

Furthermore, the present invention provides a method for producing butyric acid using SIID27451-B11 strain.

### [Effects of the Invention]

According to the present invention, a butyric acid-producing bacterium is provided that produces butyric acid more efficiently than Clostridium butyricum, which is a typical conventional butyric acid-producing bacterium, as well as more efficiently produces butyric acid compared with a type strain of Clostridium beijerinckii.

### [Brief Description of Drawing].

FIG. 1 is a photograph showing a colony image of SIID27451-B11 strain.
FIG. 2 is a photograph showing a Gram staining image of SIID27451-B11 strain
FIG. 3 shows a simple molecular phylogenetic tree based on a 16S rDNA partial sequence of SIID27451-B11 strain.

### [Description of Embodiments]

The taxonomic characteristics of SIID27451-B11 strain were examined. Hereafter, SIID27451-B11 strain will be also referred to as B11 strain. All the following tests were performed at TechnoSuruga Laboratory Co, Ltd. unless otherwise noted.

### (1) Morphological observation

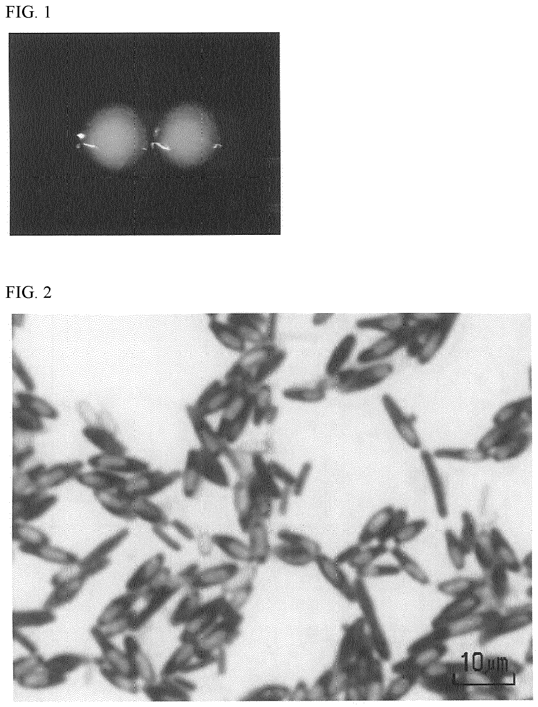

Anaerobic culture of B11 strain was performed at 30°C for 48 hours using GAM Broth "Nissui" (Nissui Pharmaceutical, Japan) + agar as a culture medium. Stereomicroscopic colony observation and Gram staining were performed to observe colonies and cell morphology of specimens. The colony and Gram staining images are shown in FIG. 1 and FIG. 2, respectively. The cell morphology was bacillus to oval bacillus (1.0-1.5 x 3.0-5.0 µm), Gram staining was positive, and spore formation was observed. Colonies were cream in color.

### (2) Physiological and biochemical properties

Anaerobic culture of B11 strain was performed at 30°C for 48 hours using GAM Broth "Nissui" (Nissui Pharmaceutical, Japan) + agar as a culture medium. An anaerobic biochemical identification kit, API20A (bioMerieux, FRA) was used to perform tests. Test items and results are shown in Table 1.

**[Table 1]**

| Table 1: Test Items and results | | |
|---|---|---|
| Test Items | Reaction / Enzyme | Results |
| IND | Indole Production^{∗} | - |
| URE | Urease^{∗} | - |
| GLU | Glucose ^{∗∗} | + |
| MAN | D-Mannitol ^{∗∗} | + |
| LAC | Lactose^{∗∗} | + |
| SAC | Saccharose^{∗∗} | + |
| MAL | Maltose^{∗∗} | + |
| SAL | Salicin^{∗∗} | + |
| XYL | D-Xylose^{∗∗} | + |
| ARA | L-Arabinose ^{∗∗} | + |
| GEL | Gelatin hydrolysis ^{∗} | - |
| ESC | Esculin hydrolysis ^{∗} | + |
| GLY | Glycerin^{∗∗} | + |
| CEL | D-Cellobiose ^{∗∗} | + |
| MNE | D-Mannose ^{∗∗} | + |
| MLZ | D-Melezitose^{∗∗} | - |
| RAF | D-Raffinose ^{∗∗} | - |
| SOR | D-Sorbitol ^{∗∗} | + |
| RHA | L-Rhamnose^{∗∗} | - |
| TRE | D-Trehalose ^{∗∗} | + |
| CAT | Catalase^{∗} | + |
| SPOR | Spore | + |
| GRAM | Gram staining | + |
| COCC | Coccus | - |

| | | |
|---|---|---|
| ^{∗}Biochemical test, ^{∗∗}Oxidation test +: Positive, -: Negative | | |

In addition, additional tests were conducted on the following items. The results are shown in Table 2 below.

**[Table 2]**

| Table 2: Additional Tests | |
|---|---|
| Test Items | Results |
| Starch hydrolysis | + |
| Growth in the presence of 5% NaCl | + |
| Growth in the presence of 6.5% NaCl | + |
| Growth in the presence of 7% NaCl | + |
| Milk coagulation test | Coagulation |

| | |
|---|---|
| +: Positive, -: Negative | |

### (3) 16S rDNA partial sequence analysis

The attribution of the specimen was inferred from the results of the analysis of 16S rDNA (16S rRNA gene) partial sequence (approximately 1500 bp) (Sequence No. 6). Anaerobic culture of B11 strain was performed at 30°C for 7 days using GAM Broth "Nissui" (Nissui Pharmaceutical, Japan) + agar as a culture medium. The 16S rDNA partial sequence analysis was performed using the following conditions.
- DNA Extraction: Achromopeptidase (FUJIFILM Wako Pure Chemical, Japan)
- PCR amplification: TKs Gflex DNA Polymerase (Takara Bio, Japan)
- Cycle Sequencing: BigDye Terminator v3.1 Cycle Sequencing Kit (Applied Biosystems, USA)
- Primer used
   PCR amplification: 9F, 1510 R
   Sequencing: 9F, 515F, 1099F, 536R, 926R, 1510R
- Sequencing: ABI PRISM 3130 xl Genetic Analyzer System (Applied Biosystems)
- Determination of base sequence: ChromasPro 2.1 (Technelysium, AUS)
- BLAST homology search:
   Analysis Software: ENKI (TechnoSuruga Laboratory, Japan)
   Database:
      DB-BA14.1 (TechnoSuruga Laboratory)
      International Nucleotide Sequence Database (DDBJ/ENA (EMBL) / GenBank) Search date: February 22, 2019
- Simple molecular phylogenetic tree analysis:
   Phylogenetic tree estimation: neighbor-joining method
   Base substitution model: Kimura-2-parameter
   Reliability evaluation of tree form: Bootstrap method (1,000 iterations)

The results are shown in Tables 3 and 4.

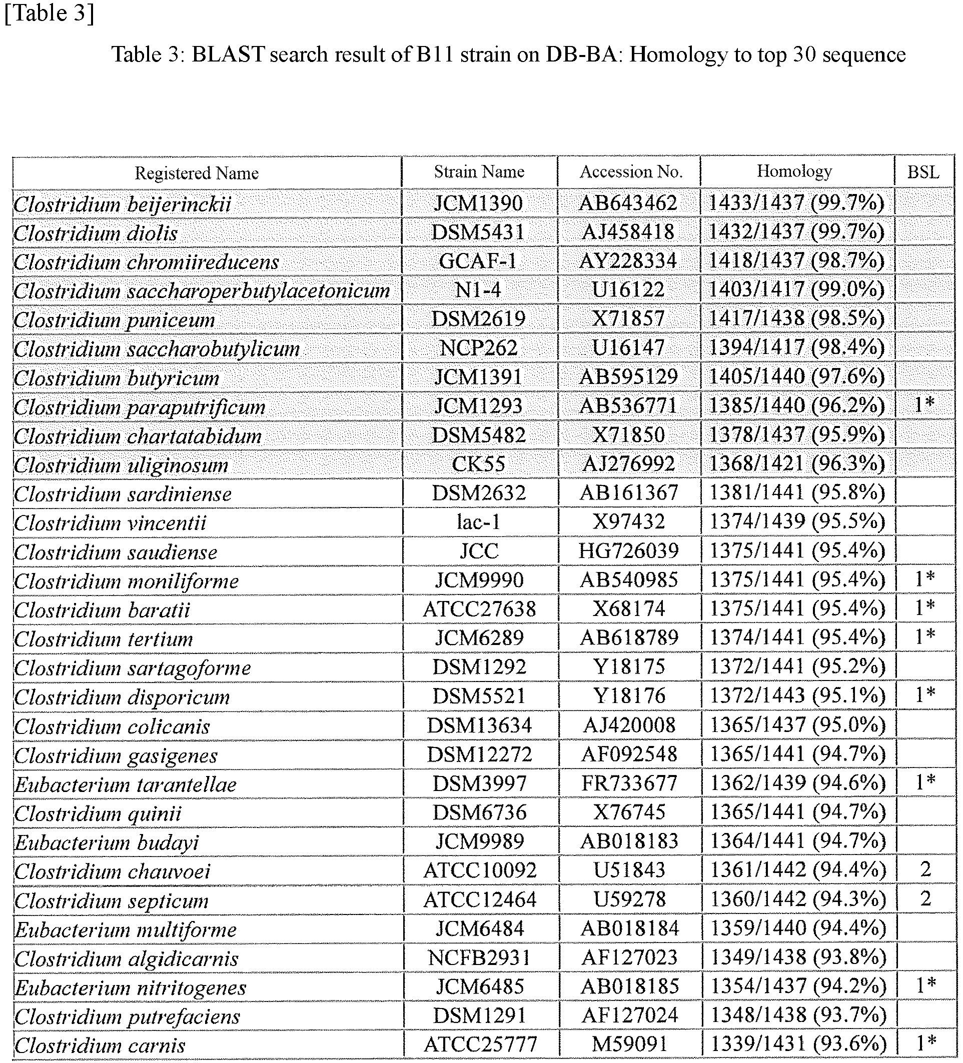

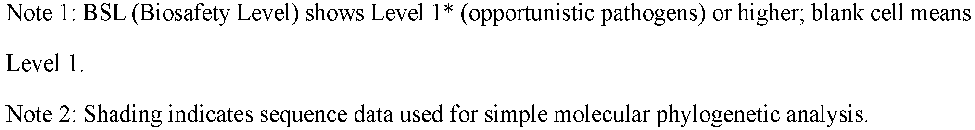

### [Table 4]

**Table 4: BLAST search result of B11 strain on International Nucleotide Sequence Database: Homology to top 30 sequence**

| Registered Name | Strain Name | Accession No. | Homology |
|---|---|---|---|
| Clostridium beijerinckii | - | LN908213 | 1435/1437 (99.9%) |
| Clostridium beijerinckii | AAU1 | KC433940 | 1435/1437 (99.9%) |
| Clostridium beijerinckii | JCM 8031 | AB678394 | 1435/1437 (99.9%) |
| Clostridium beijerinckii | JCM 7990 | AB678386 | 1435/1437 (99.9%) |
| Clostridium beijerinckii | JCM 6287 | AB640693 | 1435/1437 (99.9%) |
| Clostridium beijerinckii | JCM 7829 | LC071788 | 1435/1437 (99.9%) |
| uncultured Clostridium sp. | - | MF360200 | 1434/1437 (99.8%) |
| Clostridium beijerinckii | JCM 7837 | LC258130 | 1434/1437 (99.8%) |
| Clostridium beijerinckii | B17 | KC915012 | 1433/1435 (99.9%) |
| Clostridium beijerinckii | NRRL B-598 | CP011966 | 1434/1437 (99.8%) |
| Clostridium sp. | MF28 | CP014331 | 1434/1437 (99.8%) |
| Clostridium beijerinckii | BAS/B3/I/124 | CP016090 | 1434/1437 (99.8%) |
| Clostridium beijerinckii | B12-KKU | KT799795 | 1434/1437 (99.8%) |
| Clostridium beijerinckii | NCIMB 14988 | CP010086 | 1434/1437 (99.8%) |
| Clostridium beijerinckii | JCM 7847 | AB971810 | 1434/1437 (99.8%) |
| Clostridium sp. | G117 | JX091678 | 1434/1437 (99.8%) |
| Clostridium beijerinckii | JCM 8028 | AB678392 | 1434/1437 (99.8%) |
| Clostridium beijerinckii | JCM 8026 | AB647333 | 1434/1437 (99.8%) |
| Clostridium butyricum subsp.convexa | JCM 7840 | AB647330 | 1434/1437 (99.8%) |
| Clostridium beijerinckii | RZF1108 | GQ375085 | 1434/1437 (99.8%) |
| Clostridium beijerinckii | SA-1 | CP006777 | 1433/1437 (99.7%) |
| Clostridium beijerinckii | E102 | JX267117 | 1433/1437 (99.7%) |
| Clostridium beijerinckii | E080 | JX267098 | 1433/1437 (99.7%) |
| Clostridium beijerinckii | JCM 8025 | AB647332 | 1433/1437 (99.7%) |
| Clostridium beijerinckii | NCIMB 8052 | CP000721 | 1433/1437 (99.7%) |
| Clostridium diolis | S33-KKU | KT799798 | 1431/1434 (99.8%) |
| Clostridium beijerinckii | JCM 1390 | NR_113388 | 1433/1437 (99.7%) |
| Clostridium beijerinckii | CP23-KKU | KT799797 | 1430/1433 (99.8%) |
| Clostridium beijerinckii | E11-KKU | KT799794 | 1430/1433 (99.8%) |
| Clostridium beijerinckii | E092 | JX267108 | 1432/1437 (99.7%) |

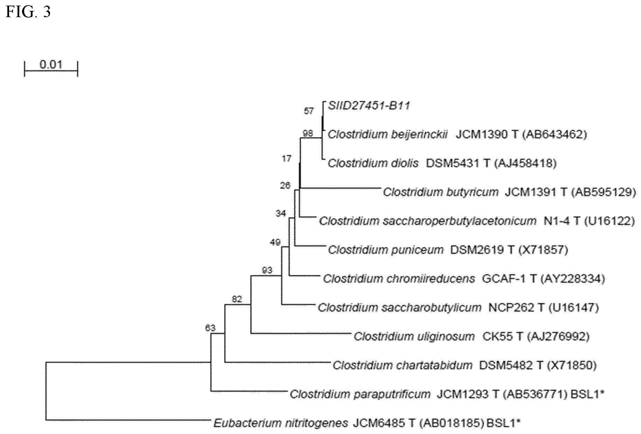

A simple molecular phylogenetic tree based on the 16S partial rDNA sequence of B 11 strain is shown in FIG. 3. In the figure, the upper left line indicates a scale bar, and 0.01 on the scale bar means that the length of the scale bar indicates 1% base difference. The numbers on the branches indicate bootstrap values, which indicates the reliability of the tree, the T at the end of the strain name indicates the Type strain of the species, and BSL indicates the biosafety level (BSL1^{∗} (opportunistic pathogen) or higher is indicated). As can be seen in FIG. 3, B11 strain is estimated to be closely related to Clostridium beijerinckii and Clostridium diolis.

### (4) Deposition of strains

Based on the above results, SIID 27451-B11 strain was found to be a strain belonging to Clostridium. SIID 27451-B11 strain was domestically deposited to National Institute of Technology and Evaluation, Patent Microorganisms Depositary (NPMD) (#122, 2-5-8, KazusaKamatari, Kisarazu-shi, Chiba 292-0818, Japan), which is an International Depositary Authority (IDA) under the terms of the Budapest Treaty, under Accession Number NITE P-02951 on May 16, 2019 and have been transferred to the international deposit on April 22, 2020 in the same institute under International Accession Numbers NITE BP-02951.
Type of microorganism: bacteria
Cell form: bacillus
Characteristics: spore-forming positive (spore-forming)
Taxonomical position: Clostridium sp.
Culture condition: GAM Broth "Nissui" + agar
Culture temperature: 30 °C
Culture period: 48 hours
Culture method: anaerobic

### (5) ANI (Average Nucleotide Identity) Analysis

Furthermore, to examine whether B11 strain is a new species, Average Nucleotide Identity (ANI) value is calculated for the top three species with the highest homology to B11 strain, namely Clostridium beijerinckii (homology 99.7%), Clostridium diolis (homology 99.7%), and Clostridium saccharoperbutylacetonicum (homology 99.0%) to determine whether the species are same or different. In ANI analysis a similarity (ANI: homology value) between a full-length genome sequence or draft genome sequence of a control strain (specimen) and a comparison strain is calculated on a computer to determine whether the species are same or different. The ANI value is calculated by using the publicly available program ANI Calculator (http://enve-omics.ce.gatech.edu/ani/index), and if the ANI value is 95% or greater, the species are determined to be the same, and if the ANI value is less than 95%, the species are determined to be different (new species).

The comparative species used in the ANI analysis are listed in Table 5 below.

### [Table 5]

**Table 5: Species used in ANI analysis**

| SIID | Species Name | Strain Name^{∗} | GenBank Accession No. |
|---|---|---|---|
| 27451-02 | *Clostridium beijerinckii* | DSM 791^{T} | GCA_002006445 |
| 27451-03 | *Clostridium diolis* | DSM 15410^{T} | GCA_008705175 |
| 27451-04 | *Clostridium saccharoperbutylacetonicum* | N1-4^{T} | GCA_000340885 |

| | | | |
|---|---|---|---|
| ^{∗} T at the end of strain name: Type strain | | | |

The results of the ANI analysis are shown in Table 6 below.

### [Table 6]

**Table 6-1: ANI values (%) between specimens**

| | SIID27451-B11 |
|---|---|
| *Clostridium beijerinckii* (SIID27451-02) | 95.81 |
| *Clostridium diolis* (SIID27451-03) | 95.76 |
| *Clostridium saccharoperbutylacetonicum* (SIID27451-04) | 82.59 |

**Table 6-2: ANI values (%) between specimens**

| | *Clostridium beijerinckii* (SIID27451-02) |
|---|---|
| *Clostridium diolis* (SIID27451-03) | 98.47 |

Since the ANI value for SIID27451-B11 strain to Clostridium saccharoperbutylacetonicum is less than 95%, SIID27451-B11 strain and Clostridium saccharoperbutylacetonicum are determined to be different species. SIID27451-B11 strain provides the ANI value of 95% or greater to Clostridium beijerinckii and Clostridium diolis, respectively. The combination of Clostridium beijerinckii and Clostridium diolis also provides the ANI value of 95% or greater. Therefore, the above three specimens are determined to be the same species. It should be noted that, according to a report of Kobayashi et al. at 2020 (Kobayashi H, et al. Int J Syst Evol Microbiol 2020, 70: 2463-2466), Clostridium diolis was shown to be the same species as Clostridium beijerinckii, and the scientific names have been integrated into Clostridium beijerinckii. Based on the above, SIID27451-B11 strain was identified as Clostridium beijerinckii.

### (6) Genome analysis

DNA of SIID27451-B11 strain was extracted and subjected to next-generation sequencing and its data analysis.

### <Extraction and purification of DNA>

DNA of B11 strain was extracted and purified using Nucleobond AXG20 column (MACHEREY-NAGEL, DE), and a purity of the extracted DNA was measured using ultra micro-volume spectrophotometer NanoDrop One (Thermo Fisher Scientific, USA). The quality inspection acceptance criteria were as follows: purity: A260 (absorbance at 260 nm, the same hereinafter)/A280 ≥ 1.8 and A260/A230 ≥ 1.6, and 20 µg (concentration of 150 ng/µL) or more. Next, fragmentation of the extracted DNA was confirmed by agarose electrophoresis. The setting conditions were 0.5% agarose, 30 V, and 1 hour. The electrophoresis results showed that, compared with the λDNA molecular weight marker (λ-Hind III digest), a band was observed around 23130 bp for the extracted DNA, and thus the extracted DNA was determined to be of good quality for use in next-generation sequencing because of its low fragmentation.

### <Next Generation Sequencing>

Next-generation sequencing and its data analysis were commissioned to Hokkaido System Science Co., Ltd.

Extracted and purified DNA samples of SIID27451-B11 strain were subjected to next-generation sequencing using next-generation sequencer PacBio RS II to obtain genome sequence information.

The genome sequence information of Clostridium beijerinckii (C. beijerinckii) type strain (GCA 002006445.1) was obtained from NCBI (National Center for Biotechnology Information, U.S.A.) web site (https://ftp.ncbi.nlm.nih.gov/genomes/all/GCA/002/006/445/GCA_002006445.1_ASM200644v1).

The genome sequences information of SIID27451-B11 and Clostridium beijerinckii type strains were auto-annotated with "DFAST", an automated annotation program for prokaryotes provided by National Institute of Genetics.

Based on the annotation information of the analyzed SIID27451-B11 and Clostridium beijerinckii type strains, draft genome sequences are compared between these two strains utilizing COG (Clusters of Orthologous Groups of protein) functional classification information. COG is a genetic function classification database for microorganisms provided by NCBI.

Table 7 shows a summary of the genomic information of the Clostridium beijerinckii type strain used, and Table 8 shows a summary of the genomic information of SIID27451-B11 strain.

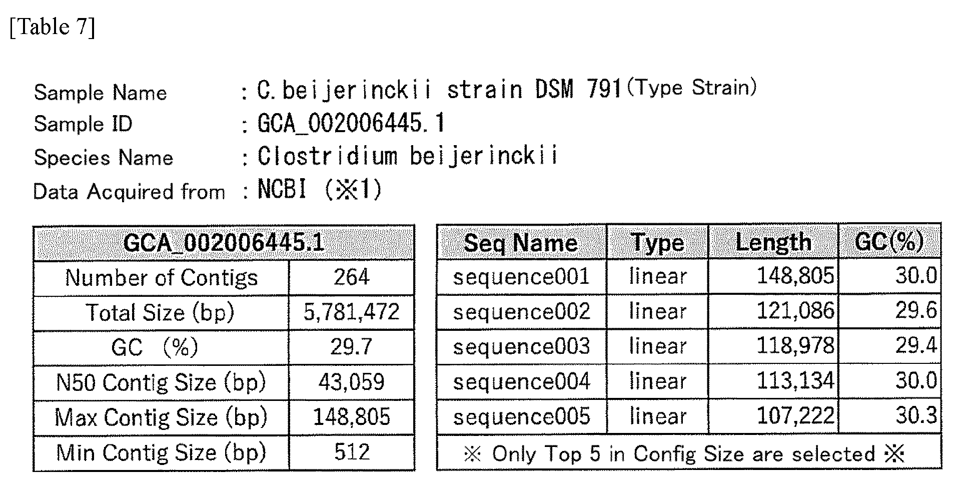

The 264 contig sequences of the Clostridium beijerinckii type strain shown in Table 7 are referred as sequence Nos. 7 to 270, respectively.

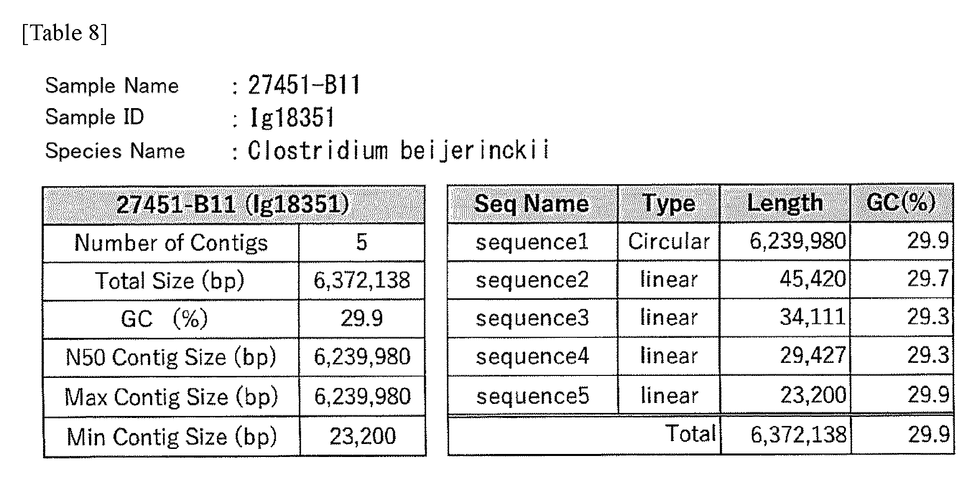

Five contig sequences 1 to 5 of SIID27451-B11 strain shown in Table 8 are referred as Sequence Nos. 1, 2, 3, 4, and 5, respectively.

Table 9 shows the predicted number of genes (ORF/rRNA/t RNA) for each strain detected by the annotation processing using DFAST.

### [Table 9]

**Table 9: Predicted number of genes for each strain**

| **Sample Name** | **Sample ID** | **ORF** | **rRNA** | **tRNA** |
|---|---|---|---|---|
| C.beijerinckii strain DSM 791 (Type Strain) | GCA_002006445.1 | 5,132 | 15 | 86 |
| 27451-B11 | Ig18351 | 5,722 | 46 | 94 |

Next, among all the ORF information detected by DFAST, we focused on the COG annotated ORF information, compared the information of the genes possessed by the type strain and B11 strain respectively, wherein the COG functional classifications of the genes have become apparent, and extracted the COG annotation information specifically detected only one of these two strains. Table 10 collectively shows the COG annotation information specifically detected for each of the Clostridium beijerinckii type strain and SIID27451-B11 strain.

[Table 10]

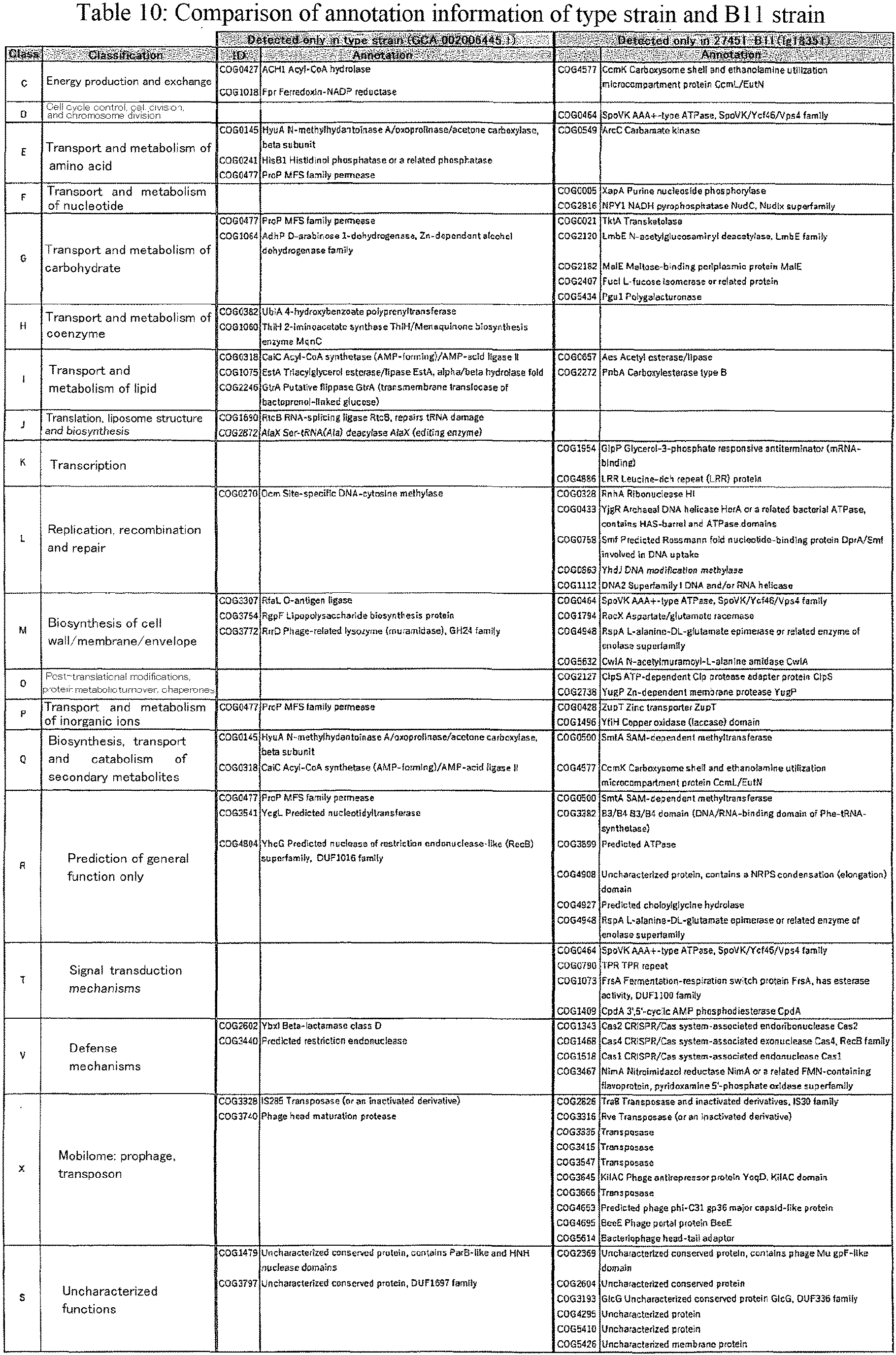

From the above results, it can be seen that the Clostridium beijerinckii type strain and SIID27451-B11 strain have sequences with different annotations. This indicates that SIID27451-B11 strain is presumed to be a strain having different characteristics from the type strain of Clostridium beijerinckii species.

As mentioned above, as a result of considering the taxonomic characterization of B11 strain, B11 strain is identified as a Clostridium beij erinckii species, while genome analysis revealed that B11 strain has sequences with different annotation from that of the type strain. Therefore, to further confirm that B11 strain is a strain with different characteristics from the type strain of Clostridium beijerinckii, we compared B11 strain, a type strain of Clostridium butyricum, which is a representative butyric acid-producing bacterium, and the type strain of Clostridium beijerinckii for their ability to produce organic acids. Details are shown in Example 1 below.

### Examples.

### Example 1: Test for organic acid-producing ability

Each strain was anaerobically cultured at 37°C for 72 hours using GAM Broth (Nissui Pharmaceutical, Japan) as a culture medium. The culture solution was filtered through a membrane filter with a pore size of 0.20 µm and used as a sample solution. The concentration of organic acids in the sample solution was determined by high-performance liquid chromatography. This test was conducted on February 13, 2020, at T TechnoSuruga Laboratory Co, Ltd. The measurement conditions were as follows:
- System: Shimadzu Organic Acid Analysis System (Shimadzu, Japan)
- Columns: Shim-pack SCR-102(H) 300mm x 8mm ID, 2 columns in series
- Guard columns: Shim-pack SCR-102(H) 50mm x 6mm ID
- Eluent: 5 mmol/L of p-toluene sulfonic acid
- Reaction solution: 5 mmol/L of p-toluene sulfonic acid, 100 µmol/L of EDTA, and 20 mmol/L of Bis-Tris
- Flow rate: 0.8 mL/min
- Oven temperature: 45°C
- Detector: Conductivity Detector CDD-10A

The nine organic acids measured were succinic acid, lactic acid, formic acid, acetic acid, propionic acid, iso-butyric acid, n-butyric acid, iso-valeric acid, and n-valeric acid. The results are shown in Table 11 below.

### [Table 11]

**Table 11: Test for organic acid-producing ability**

| (µg/mL) | | | | | | | | | |
|---|---|---|---|---|---|---|---|---|---|
| Sample name | succinic acid | acetic acid | formic acid | acetic acid | propioni c acid | iso-butyric acid | n-butyric acid | iso-valeric acid | n-valeric acid |
| Control (medium only) | 163 | 131 | 30 | 164 | 63 | | | | |
| Clostridium butyricum NBRC 13949T | 158 | 1107 | 804 | 1147 | 62 | | 1030 | | |
| Clostridium beijerinckii NBRC 109359T | 161 | 1038 | 26 | 1189 | 63 | | 1530 | | |
| 27451-B11 | 161 | | 49 | 240 | 69 | | 2828 | | |

Blank cells in the table are below the lower limit of quantitation. The lower limit of quantification is 5 µg/mL for succinic acid, lactic acid, acetic acid, and propionic acid, and 10 µg/mL for formic acid, iso -butyric acid, n-butyric acid, iso-valeric acid, and n-valeric acid. The values for propionic acid include foreign substances.

It can be seen that B11 strain produced 2.74 times more n-butyric acid than Clostridium butyricum, which is a well-known butyric acid bacterium. Furthermore, it can be seen that B11 produced almost exclusively butyric acid when the amounts of organic acids contained in the medium (Control) are subtracted. In contrast, Clostridium butyricum produced lactic acid, formic acid, and acetic acid in addition to butyric acid. This means that B11 strain may be a very efficient and selective butyric acid-producing bacterium. In particular, B11 produced very little formic acid. Formic acid may have a deleterious effect on humans. Since B11 produces very little the formic acid, it is expected to be used for biological applications.

In addition, it can be seen that B11 strain produced different types and amounts of organic acids compared with Clostridium beijerinckii NBRC 109359T, the type strain of Clostridium beijerinckii. B11 strain produced lactic acid below the limit of quantification, less acetic acid, and more butyric acid compared with the type strain, Clostridium beijerinckii NBRC 109359T. Therefore, it has been determined that B11 strain is a strain having different characteristics from the type strain of Clostridium beijerinckii species.

### [References to deposited biological material]

(1) Name of depositary: National Institute of Technology and Evaluation, Patent Microorganisms Depositary
(2) Contact: #122, 2-5-8, KazusaKamatari, Kisarazu-shi, Chiba 292-0818, Japan,
   Telephone No. 0438-20 -5580
(3) Accession number: NITE BP- 02951
(4) Indication reference: SIID27451-B11
(5) Date of original deposit date: May 16, 2019

### [Sequence listing]

HM06P-1_ST25.txt

## Claims

1. Clostridium beijerinckii SIID27451-B11 strain (accession number NITE BP-02951).

2. Clostridium beijerinckii SIID27451-B11 strain according to claim 1, having genome sequences shown in sequence numbers 1 to 5.

3. Clostridium beijerinckii SIID27451-B11 strain according to claim 1, having a 16S rDNA sequence shown in sequence number 6.

4. Clostridium beijerinckii SIID27451-B11 strain according to claim 1, which produces more butyric acid and less lactic acid and acetic acid in a culture solution after anaerobically culturing the strain at 37 °C for 72 hours, compared with Clostridium beijerinckii NBRC 109359T, which is a type strain of Clostridium beijerinckii.

5. A method for producing butyric acid using SIID27451-B11 strain according to any one of claims 1 to 4
